# EUROPEAN PATENT APPLICATION

(11) **EP 0 853 087 A1**
(43) Date of publication of application: **15.07.1998**
(21) Application number: 98100234.8
(22) Date of filing: 08.01.1998
(51) Int. Cl.: C07H 17/08

(54) **A process for the purification of erythromycin**

(30) Priority: 10.01.1997 GB 9700410
(71) Applicant: Biochemie S.A., 08400 Granollers/Barcelona (ES)
(72) Inventor: Diago, Jose C., 08400 Granollers, Barcelona (ES); Centellas, Victor, 08440 Cardedeu, Barcelona (ES)
(74) Representative: Ross, Richard Anthony Mabyn

(57) **Abstract**

A process for the purification of impure erythromycin in form of a thiocyanate, which comprises treating impure erythromycin in form of a thiocyanate in non-halogenated solvent and separating off purified erythromycin in form of a thiocyanate; an erythromycin A composition which is free of halogenated solvent and wherein erythromycin A is in form of a thiocyanate, containing 3% or less of erythromycin B and, for example 65% and more of erythromycin A in form of a thiocyanate and its use in the production of erythromycin in free base form of high quality.

## Description

The present invention relates to a process for purifying impure erythromycin, as *e.g.* obtained in a fermentation broth.

Erythromycin which may *e.g.* be produced by *Streptomyces erythreus*, is a known compound and known to have useful pharmaceutical, *e.g.* antibiotic properties, *e.g.* therapeutic properties. Erythromycin is described to show, *e.g.* a broad activity range and low toxicity and may be useful as a pharmaceutical.

Erythromycin may be a mixture of compounds of formula 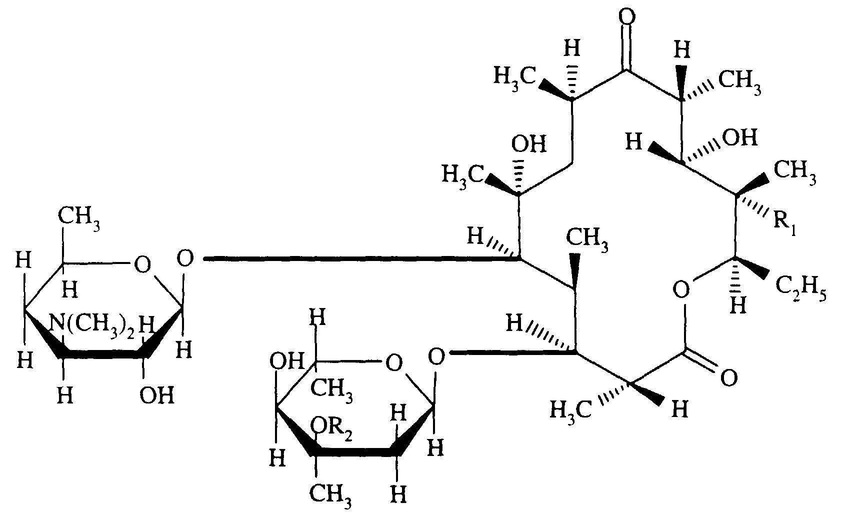 wherein R₁ and R₂ may have different meanings, *e.g.* the main component of such a mixture may be erythromycin A, *e.g.* a compound of formula I, wherein R₁ is hydroxy and R₂ is methyl. Commercially available erythromycin may contain mainly erythromycin A which may include, *e.g.* a content, of other erythromycins, such as erythromycin B of formula I, wherein R₁ is hydrogen and R₂ is methyl; and, *e.g.* erythromycin C of formula I, wherein R₁ is hydroxy and R₂ is hydrogen. A fermentation broth obtainable from a fermentation process in the production of erythromycin may *e.g.* contain up to 10% and more erythromycin B.

Erythromycin, as used herein, is meant to be a mixture of erythromycin A, B and C, if not otherwise stated.

Processes for erythromycin production, *e.g.* via fermentation, are known and erythromycin isolation from the fermentation broth may *e.g.* be based on
- adsorption of erythromycin in the fermentation broth onto adsorption resins, see for example Chemical Abstracts Vol. 116:82111 and Chemical Abstracts Vol. 81:118537 (CZ 152,850);
- solvent extraction at *e.g.* basic pH from an aqueous fermentation broth, as for example disclosed in Chemical Abstract Vol. 73: 129577 (DD 72,351); Chemical Abstracts Vol. 74:52145 (PL 60,228); Chemical Abstracts Vol. 101:157602 (Khim.-Farm. Zh. 1974, 8(8), 44-6); Chemical Abstract Vol. 88: 188135 (PL 87,598); Chemical Abstracts Vol. 119:158376 (HU 62,938); Derwent Abstract No. 76-38164X (CA 988514); Chemical Abstracts Vol. 78:109318 (GB 1,301,389); Chemical Abstract Vol. 90: 119759 (ES 454,269); and Chemical Abstracts Vol 53:6549 (US 2,864,817);
   according to which an enriched erythromycin extract may be obtained by extraction of an erythromycin containing fermentation broth with water-immiscible solvents; such as amyl acetate, butyl acetate, chloroform, methylene chloride, methylisobutylketone and xylene; and precipitation of erythromycin in form of an acid-addition salt, such as in form of an acetate, citrate, lactate, oxalate, perchlorate, salycilate, sulfamate, tartrate and thiocyanate salt.
Processes for purification of an erythromycin salt, e.g. a thiocyanate salt may *e.g.* be based on the isolation of an acid addition salt of erythromycin, obtainable as described above; and conversion of that salt into erythromycin in free base form; *e.g.* by addition of a base; *e.g.* to an aqueous suspension of an erythromycin salt; and optionally extracting erythromycin in free base form into an organic solvent; such as chlorinated hydrocarbon, *e.g.* methylene chloride, as for example described in Chemical Abstracts 95:22940 (RO 67,214), Chemical Abstracts Vol. 73:129577 (DD 72,351) and Chemical Abstracts Vol. 121, 205893 (RO 103274); In Chemical Abstracts Vol. 76:99997 (US 3,637,654) a purification of an erythromycin thiocyanate in trichloroethylene is described.

According to Chemical Abstract Vol. 84:162890 (PL 76,648) and Chemical Abstracts 114:43482(JP 02,196,796) erythromycin may be isolated from an aqueous acidic medium; but the yields may be low and the final product may be contaminated, *e.g.* with a by-product of formula 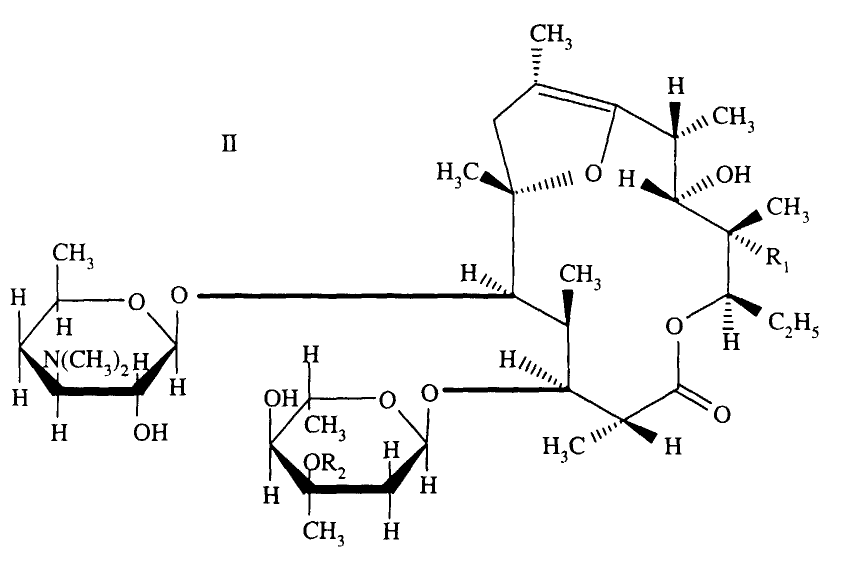 or similar, related compounds, *e.g.* in an amount of ca. 1 to 2%.

Thus, according to known processes in the production of purified erythromycin,
- erythromycin is isolated from an acidic aqueous solvent (system) which may result in low yields and the formation of by-products;
- erythromycin is obtained in form of an acid addition salt, for example in form of a salt with a thiocyanic acid and transformed into purified erythromycin by use of chlorinated hydrocarbons.
Erythromycin, containing mainly erythromycin A, which is commercially available may thus be contaminated with chlorinated hydrocarbons, or, it may have a content of by-products, for example of formula II or a similar, related compound, e.g. more than 200 ppm.

Surprisingly it was now found that erythromycin, *e.g.* in form of a salt with a thiocyanic acid, may be isolated from the fermentation broth and purified without using halogenated solvent in high quality and yield, *e.g.* having a high content of erythromycin A and a small content of erythromycin B, and lacking by-products, such as of formula II. Purified erythromycin in form of a salt with a thiocyanic acid may be converted into purified erythromycin in free base form.

In one aspect the present invention provides a process for the purification of impure erythromycin in form of a thiocyanate, which comprises treating impure erythromycin in form of a thiocyanate in non-halogenated solvent and separating off purified erythromycin in form of a thiocyanate.

A compound containing erythromycin and a thiocyanic acid, *e.g.* a salt of erythromycin with a thiocyanic acid, is hereinafter designated as "erythromycin in form of a thiocyanate", or, abbreviated, "ERYTH" (erythromycin A in form of a thiocyanate as "ERYTH A"). ERYTH may be in free form or in form of a hydrate, *e.g.* in form of a dihydrate. Thiocyanic acid is believed to be a tautomeric mixture of HSCN and HNCS (isothiocyanic acid).

Impure erythromycin, *e.g.* erythromycin A, *e.g.* in form of a thiocyanate, may *e.g.* have a higher content of erythromycin B, than purified erythromycin, e.g. erythromycin A, *e.g.* in form of a thiocyanate.

Erythromycin may be extracted from a fermentation broth, optionally from a pre-treated fermentation broth, *e.g.* as appropriate, *e.g.* as conventional, *e.g.* by use of an organic solvent. Pre-treatment includes for example removing at least pants of the solids of the fermentation broth, *e.g.* as appropriate, *e.g.* as conventional, for example by flocculation, decantation, filtration, centrifugation; charcoal treatment; pre-concentration; pre-extraction. An, *e.g.* organic, extract containing erythromycin may be treated, for example as appropriate, *e.g.* as conventional, with, for example an acid, *e.g.* with thiocyanic acid and erythromycin in form of a thiocyanate, *e.g.* ERYTH, may be formed, precipitated and isolated, for example as appropriate, *e.g.* as conventional.

The present invention may be carried out as follows:
Impure erythromycin, *e.g.* in solid form, in form of a thiocyanate, containing *e.g.* mainly erythromycin A, obtained *e.g.* as appropriate, *e.g.* as conventional, *e.g.* from a fermentation broth, *e.g.* as appropriate, *e.g.* as conventional, such as described above, containing as an impurity *e.g.* erythromycin B, is treated in non-halogenated solvent. The term non-halogenated solvent used herein is understood to be an organic solvent, the chemical formula of which does not contain halogen atoms. In contrast to this, halogenated solvent as used herein is understood to be a an organic solvent, the chemical formula of which does contain at least one halogen atom.
Non-halogenated solvent includes solvent which is soluble or insoluble in water, including *e.g.* a solvent of a low dielectric constant, *e.g.* appropriate ketones, such as methyl-(C₁₋₅)alkyl-ketones, *e.g.* acetone or methylisobutyl ketone; di-(C₂₋₅)alkyl ketones, such as diethyl ketone or diisopropylketone; esters, such as (C₁₋₆)alkanoic acid, *e.g.* (C₁₋₄)alkanoic acid, *e.g.* acetic acid alkyl esters, wherein alkyl is of C₁₋₈, such as C₁₋₆, e.g. C₁₋₄, for example butylacetate or amylacetate; and aromatic hydrocarbons, such as xylene; and solvents of higher dielectric constant, e.g. alcohols, such as (C₁₋₅)alcohols, for example methanol and isopropanol; and amides, such as (C₁₋₄)monoalkyl amides, e.g. N-methylformamide and (C₁₋₄)dialkyl amides, such as dimethylacetamide and dimethylformamide; preferably ketones, such as methyl-(C₁₋₅)alkyl-ketones; esters, such as acetic acid (C₁₋₈)alkyl esters; and alcohols; *e.g.* acetone, methylisobutylketone, methyl acetate, ethyl acetate and methanol.
The solvent may be a solvent system and may contain more than one solvent and water. The water content is not critical. It is, however, preferred that the water content is small. The water content may be for example about 1% to about 20%, e.g. 1% to 20%, such as about 3% to about 15%, e.g. 3% to 15%, such as about 5% to about 10%, for example 5% to 10%, e.g. about 6% to about 8%, e.g. 6% to 8%.
An alcohol, such as methanol and an ester, such as methyl acetate or ethyl acetate may be used preferably in the presence of water.
The organic non-halogenated solvent may be used in an appropriate amount in respect to the amount of impure ERYTH. For example about 1.5 ml to 50 ml and more solvent per gram of erythromycin in form of a thiocyanate may be used. Preferably a low amount of solvent may be used, *e.g.* about 1.5 ml up to 35 ml and more, for example 1.5 ml to 35 ml; such as about 2.0 ml to about 30 ml, for example 2.0 ml to 30 ml; such as about 2.5 ml to about 20 ml, for example 2.5 ml to 20 ml; per gram of ERYTH.
In another aspect the present invention provides a process for the purification of impure erythromycin in form of a thiocyanate, which comprises treating impure erythromycin in form of a thiocyanate in an ester, a ketone or an alcohol and separating off purified erythromycin in form of a thiocyanate.

Treatment of impure erythromycin, *e.g.* erythromycin A in form of a thiocyanate includes suspending or dissolving, preferably suspending, impure ERYTH in non-halogenated solvent, for example at temperatures from about -50° C to the boiling point of the solvent (system), preferably from about 0° C to about 80° C, for example 0° C to 80° C, e.g. about 70° C to about 70° C, such as 40° C to 70° C.
An acid, such as acetic acid, hydrochloric acid and sulphuric acid, or a base, such as triethylamine, ammonia or sodium hydroxide may be added to keep the pH in an appropriate range. Impure ERYTH may be dissolved, *e.g.* as appropriate, *e.g.* as conventional, *e.g.* by addition of a base and ERYTH may be reprecipitated, *e.g.* as appropriate, *e.g.* as conventional, such as by addition of thiocyanate-ions from an appropriate source or by crystallisation from the ERYTH containing solution.
In the preferred case of suspending impure ERYTH, a suspension of ERYTH may be treated, *e.g.* agitated, for example stirred, *e.g.* for some time, *e.g.* until a purification of ERYTH is obtained, for example stirrred for ca. 30 minutes and more, *e.g.* about 30 minutes to 5 hours, such as about 30 minutes to 3 hours.
The suspension of ERYTH in non-halogenated solvent may be concentrated, for example prior to isolation, *e.g.* as appropriate, *e.g.* as conventional, *e.g.* by distilling off at least parts of the non-halogenated solvent, if desired.

In another aspect the present invention provides a process as described above, wherein impure erythromycin in form of a thiocyanate is suspended and agitated in non-halogenated solvent and purified erythromycin in form of a thiocyanate is separated off.

Purified erythromycin in form of a thiocyanate may be isolated by seperating off purified ERYTH after treatment from the non-halogen containing solvent used, *e.g.* as appropriate, *e.g.* as conventional, *e.g.* by filtration or centrifugation.

Purified erythromycin in form of a thiocyanate obtained according to the process of the present invention may be, *e.g.* free of halogenated solvent. Purified ERYTH may have a content of erythromycin A in form of the thioyanate of 65% and more (no water correction), for example 75% and more; such as about 65% to about 80%; such as 65% to 79%. Purified ERYTH A may contain erythromycin B in an amount of 3%, preferably less than 3%, for example 2% and even less, such as 1% and even less, *e.g.* up to 3%, 2% or 1%, e.g. 0.8%. It may contain small amounts of non-halogenated solvents, detectable, for example, by gas chromatography.

An erythromycin A composition in form of a thiocyanate containing up to 3% erythromycin B which is free of halogenated solvent is new and also forms part of the present invention.

In another aspect the invention provides an erythromycin A composition which is free of halogenated solvent and wherein erythromycin A is in form of a thiocyanate, containing 3% or less erythromycin B; and containing, for example, 65% and more of erythromycin A in form of a thiocyanate.

An erythromycin composition in form of a thiocyanate, obtainable according to the present invention, is useful, for example for the conversion of purified ERYTH into an erythromycin composition wherein erythromycin is in form of the free base, of high quality, for example having a low content of erythromycin B and a high content of erythromycin A, which is free of halogenated solvents without further purification steps.

Purified erythromycin in form of a thiocyanate may be converted into erythromycin in form of the free base as appropriate, for example, as appropriate, *e.g.* as conventional. Non-halogenated solvent is used. Conversion may he carried out, for example, in similar manner, for example in analogous manner as described, *e.g.* in US 2,864,817.

Conversion may be carried out, for example, by suspending purified ERYTH, obtainable according to the present invention, in water and adjusting the pH as appropriate; e.g. to a pH at which erythromycin may be isolated in free base form; or *e.g.* treating purified ERYTH, in aqueous or anhydrous, non-halogenated organic solvent and adjusting the pH as appropriate, if desired. An appropriate pH-range may be, *e.g.* from about 9.0 to about 11.0, such as 9.0 to 11.0; for example from about 9.5 to about 10.5, for example 9.5 to 10.5. Adjustment of the pH may be effected as appropriate, *e.g.* as conventional, *e.g.* by addition of a base, for example a hydroxide, such as sodium hydroxide.
Preferably water, or a mixture of water and an organic, non-halogenated solvent, e.g. such as described above, for example a ketone, an ester or an alcohol, *e.g.* a ketone or an alcohol, may be used. Preferably the organic solvent is water-miscible. Preferred organic solvents include acetone and methanol. The organic solvent may be used in small amounts.
The mixture may be stirred for some time and a precipitate of erythromycin in form of a free base may be obtained and separated off, *e.g.* as appropriate, *e.g.* as conventional, such as by filtration or centrifugation

In another aspect the present invention provides a process for the production of erythromycin in form of the free base having a high content of erythromycin A and a low content of erythromycin B which comprises
(i) treating impure erythromycin in form of a thiocyanate in non-halogenated solvent and separating off purified erythromycin in form of a thiocyanate, and
(ii) converting purified erythromycin in form of a thiocyanate obtainable in step (i) into erythromycin in form of the free base in non-halogenated solvent.

In a preferred embodiment of the present invention impure ERYTH may be suspended in a non-halogenated solvent at a temperature from about 0° C to about 50° C and, after a short period of agitating, *e.g.* stirring, purified ERYTH may be isolated, for example by filtration. The suspension may be concentrated, for example prior to filtration, by distilling off at least pants of the solvent. From mother liquors obtained further ERYTH may be obtained, e.g. as appropriate, *e.g.* as conventional, for example by addition of water to the mother liquors obtained after the filtration of the purified ERYTH, and stripping off the organic solvent system used in purification. Purified ERYTH, *e.g.* as obtainable according to the present invention may be converted into erythromycin in free base form, e.g. as appropriate, *e.g.* as conventional, for example suspending purified ERYTH in a mixture of water, if desired in the presence of a small amount of an organic solvent, such as acetone or methanol. The pH may *e.g.* be adjusted, *e.g.* to obtain 9.5 to 10.5, *e.g.* as appropriate, *e.g.* as conventional, such as by addition of a solution of a base, *e.g.* sodium hydroxide. Erythromycin in free base form may be separated off, *e.g.* by filtration.
Erythromycin in form of the free base may be obtained according to the present invention from purified ERYTH in high yields, *e.g.* 95% and more, even 98% and more, containing, for example, 96% and more, *e.g.* 98% and more of erythromycin A (on an anhydrous basis). The content of erythromycin B in erythromycin in form of the free base obtained according to the present invention may be about the same as in purified ERYTH, e.g. purified according to the present invention.

The overall yields in the produciton of erythromycin in form of the free base, starting from impure ERYTH, may be higher than 85%, for example higher than 90% (depending on the content of erythromycin in the crude ERYTH). In erythromycin in free base form obtained according to the present invention the content of erythromycin A (on an anhydrous basis) may he, for example 96% and more, for example 98% and more. The content of erythromycin B may be 3% and less, for example 2% and less, *e.g.* 1% and less, such as 0.8%.

The process according to the present invention has a number of significant practical and economical advantages and is useful *e.g.* on industrial scale. Purification of erythromycin in form of ERYTH including, for example, reduction of the content of erythromycin B, can be easily and ecologically carried out and the purified erythromycin in form of ERYTH may be easily and ecologically converted into erythromycin in form of the free base, which is free of halogenated solvents, in an overall almost quantitative yield. Erythromycin in form of the free base obtained according to the present invention may contain more than 96% erythromycin A and less than 1% of erythromycin B.

The process of the present invention is thus useful in the purification of erythromycin, *e.g.* in the purification of ERYTH, obtainable *e.g.* from an erythromycin containing fermentation broth; and in production of erythromycin in free base form in high quality, containing e.g. a high amount of erythromycion A and a low amount of erythromycin B.

In another aspect the present invention provides the use of an erythromycin composition which is free of halogenated solvent and wherein erythromycin A is in form of a thiocyanate, containing 3% or less of erythromycin B and *e.g.* containing more than 65% of erythromycin A in form of a thiocyanate in the production of erythromycin in free base form.

In another aspect the present invention provides the use of non-halogenated solvent in the purification of impure erythromycin in form of a thiocyanate.

The production of any starting material used according to the process of the present invention, e.g. ERYTH, which is not particularly described herein may be carried out as appropriate, for example as appropriate, *e.g.* as conventional, for example in analoguous manner to conventional processes.

The following examples illustrate the invention. All temperatures are in degree Celsius and are uncorrected.
The content of erythromycin A and B (ASSAY and B-CONTENT) is determined by liquid chromatography (HPLC).

### Abbreviations:

- ERYTH:: Erythromycin in form of a thiocyanate (optionally in a hydrate form, such as a dihydrate)
- ASSAY:: Content of erythromycin A (no water correction)
- B-CONTENT:: Content of erythromycin B
- YIELD 1:: Yield (%) after filtration of the precipitate (ERYTH) treated with non-halogenated solvent. YIELD 1 is corrected by ASSAY.
- YIELD 2:: Overall yield (%) starting from impure ERYTH; including yield from mother liquors; obtainable by addition of water to the mother liquors and distilling off organic solvent.

The examples illustrate in step (i) the purification of ERYTH (EXAMPLES 1 to 14) and in step (ii) conversion of purified ERYTH into erythromycin in free base form.

### EXAMPLES 1 to 4

ASSAY of ERYTH-starting material: 63.4%; B-CONTENT: 4.4%.
(i) 100 g of ERYTH , dry or wet, are suspended in V ml of methylisobutylketone. The suspension is stirred for ca. 60 minutes at T ° C, purified ERYTH is filtrated off and washed with the same solvent (*e.g.* about 20% of V). The results obtained are shown in Table 1 below.

**Table 1**

| T (°C) | ASSAY | B-CONTENT | YIELD 1 | YIELD 2 | WEIGHT (g) | V (ml) |
|---|---|---|---|---|---|---|
| 55 | 66.9 | 2.8 | 88 | 95 | 83.3 | 1000 |
| 45 | 71.6 | 2.3 | 83 | 98 | 73.6 | 2000 |
| 65 | 68.0 | 3.1 | 88 | 91 | 81.8 | 1000 |
| 45 | 68.1 | 3.4 | 95 | 98 | 88.4 | 1000 |

### EXAMPLE 5

B-CONTENT of ERYTH-starting material: 4.8%.
(i) 100 g of ERYTH, dry or wet, are suspended in a mixture of 900 ml of methyl acetate and 100 ml of water. The suspension is stirred for ca. 120 minutes at ca. 30/35° C, purified ERYTH is filtrated off and washed with the same solvent system. 84.8 g i.e. 95.0% of theory, of purified ERYTH are obtained. B-CONTENT: 2.4%.

### EXAMPLE 6

B-CONTENT of ERYTH-starting material: 4.8%.
(i) 100 g of crude ERYTH , dry or wet, are suspended in a mixture of 900 ml of ethyl acetate and 100 ml of water. The suspension is stirred for ca. 120 minutes at ca. 30/35° C, purified ERYTH is filtrated off and washed with the same solvent system. 94.2 g, i.e. 98.0% of theory, of purified ERYTH are obtained. B-CONTENT: 3.9%.

### EXAMPLES 7 to 10

ASSAY of ERYTH-starting material: 65.2%; B-CONTENT: 4.9%.
(i) 100 g of crude ERYTH , dry or wet, are suspended in 1000 ml of aqueous methanol having different % of water (see %H₂O in Table 2). The suspension is stirred for ca. 60 minutes at T °C, purified ERYTH is filtrated off and washed with the same solvent system. The results obtained are shown in Table 2 below.

**Table 2**

| T (°C) | ASSAY | B-CONTENT | YIELD 1 | YIELD 2 | WEIGHT (g) | % H₂O |
|---|---|---|---|---|---|---|
| 32 | 70.4 | 3.7 | 98 | 99 | 90.3 | 80 |
| 32 | 74.8 | 2.9 | 90 | 98 | 78.5 | 60 |
| 40 | 74.9 | 3.5 | 98 | 99 | 88.4 | 80 |
| 40 | 78.1 | 2.1 | 92 | 94 | 76.5 | 60 |

### EXAMPLES 11 to 14

ASSAY of ERYTH-starting material: 70.7%; B-CONTENT: 5.4%.
(i) 100 g of crude ERYTH , dry or wet, are suspended in V ml of acetone (see Table 3). The suspension is stirred for ca. 120 minutes at T °C, purified ERYTH is filtrated off and washed with the same solvent system. The results obtained are shown in Table 3 below.

**Table 3**

| T (°C) | ASSAY | B-CONTENT | YIELD 1 | YIELD 2 | WEIGHT (g) | V |
|---|---|---|---|---|---|---|
| 32 | 76.9 | 0.8 | 68 | 90 | 62.9 | 1000 |
| 42 | 78.1 | 1.0 | 83 | 93 | 75.0 | 500 |
| 32 | 78.9 | 1.6 | 87 | 98 | 77.8 | 500 |
| 37 | 78.9 | 1.6 | 89 | 96 | 79.3 | 250 |

### EXAMPLE 15

(ii) 12 g of ERYTH, purified in step (i), for example as described in any of Examples 1 to 14, are suspended in a mixture of 120 ml of water and. A small amount of acetone or methanol may be added. The suspension is stirred for ca. 2 hours at ca. 40 to 50°C and the pH is adjusted to ca. 9.5 to 10.5 by addition of a solution of sodium hydroxide. Erythromycin in free base form is filtrated off and washed with hot water.
   YIELD 1: higher than 95%
   YIELD 2: up to 98%
   ASSAY: higher than 96%
   B-CONTENT: practically identical to purified ERYTH-starting material, obtained according to Examples 1 to 14.

## Claims

1. A process for the purification of impure erythromycin in form of a thiocyanate, which comprises treating impure erythromycin in form of a thiocyanate in non-halogenated solvent and separating off purified erythromycin in form of a thiocyanate.

2. A process according to claim 1, wherein a non-halogenated solvent is a ketone, an ester or an alcohol.

3. A process according to any one of claim 1 to 2, wherein impure erythromycin in form of a thiocyanate is suspended and agitated in non-halogenated solvent and purified erythromycin in form of a thiocyanate is separated off.

4. Erythromycin A composition which is free of halogenated solvent and wherein erythromycin A is in form of a thiocyanate, containing 3% or less of erythromycin B.

5. Erythromycin composition according to claim 4, containing 65% and more of erythromycin A in form of a thiocyanate.

6. A process for the production of erythromycin in form of the free base having a high content of erythromycin A and a low content of erythromycin B which comprises
(i) treating impure erythromycin in form of a thiocyanate in non-halogenated solvent and separating off purified erythromycin in form of a thiocyanate, and
(ii) converting purified erythromycin in form of a thiocyanate obtainable in step (i) into erythromycin in form of the free base in non-halogenated solvent.

7. Use of an erythromycin composition according to anyone of claims 4 to 5 in the production of erythromycin in free base form.

8. Use of non-halogenated solvent in the purification of impure erythromycin in form of a thiocyanate.
